(19) Europäisches Patentamt
European Patent Office
Office européen des brevets

(11) Publication number: 0 234 752 A1

(12) EUROPEAN PATENT APPLICATION

(21) Application number: 87300733.0

(22) Date of filing: 28.01.87

(51) Int. Cl.4: A61K 31/365 , A01N 43/20 , //C07D305/12

The applicant has filed a statement in accordance with Rule 28 (4) EPC (issue of a sample only to an expert). Accession number(s) of the deposit(s): ATCC 20788, ATCC 20789, ATCC 20790.

(30) Priority: 03.02.86 US 825496

(43) Date of publication of application: 02.09.87 Bulletin 87/36

(84) Designated Contracting States: CH DE FR GB IT LI NL

(71) Applicant: MERCK & CO. INC.
126, East Lincoln Avenue P.O. Box 2000
Rahway New Jersey 07065(US)

(72) Inventor: Garrity, George M.
617 Clark Street
Westfield New Jersey 07090(US)
Inventor: Onishi, Janet C.
350 Cherry Hill Road
Mountainside New Jersey 07092(US)
Inventor: Del Val, Sagrario M.
Pez Volador - 14
Madrid 30(ES)

(74) Representative: Hesketh, Alan, Dr.
European Patent Department Merck & Co., Inc. Terlings Park Eastwick Road
Harlow Essex, CM20 2QR(GB)

(54) **Antifungal beta-lactones.**

(57) The compounds of the following general structural formula (I)

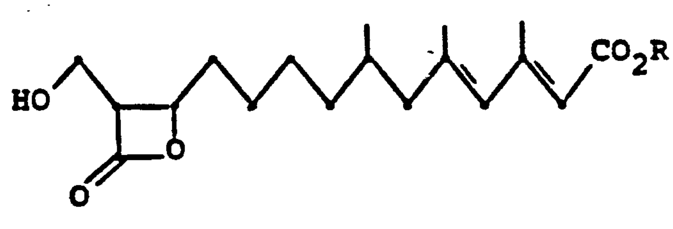

(I)

wherein R is hydrogen or $C_{1-3}$alkyl and the tetrahydro analogs thereof, are disclosed.

The compounds are isolated from a fermentation broth of known fungus and esterification of the product produced thereby. The compounds have been found to have significant antifungal activity.

EP 0 234 752 A1

# ANTIFUNGAL β-LACTONES

## BACKGROUND OF THE INVENTION

The compound of the formula (I), wherein R is hydrogen, I2-hydroxy-I3-hydroxymethyl-3,5,7-trimethyltetradeca-2,4-dienoic acid I2,I4-lactone, was identified as an antibiotic fungal metabolite in I970 [Aldridge et al., Chem. Comm., I970, p. 639]. The compounds of the formula (I), wherein R is methyl and the tetrahydro analog wherein R is hydrogen were disclosed in the structure elucidation of the compound of the formula - (I) wherein R is hydrogen [Alridge et al. J. Chem. Soc. (C), I97I, pp. 3888-389I].

## SUMMARY OF THE INVENTION

This invention relates to the novel pharmacological properties of the compounds of the formula (I) which have been found to be antifungal agents.

## DETAILED DESCRIPTION OF THE INVENTION

The present invention is directed to a method of inhibiting fungal growth which comprises the administration to a subject in need of such treatment a nontoxic therapeutically effective amount or applying to a surface requiring control of fungal growth an antifungally effective amount of a compound represented by the following general structural formula (I)

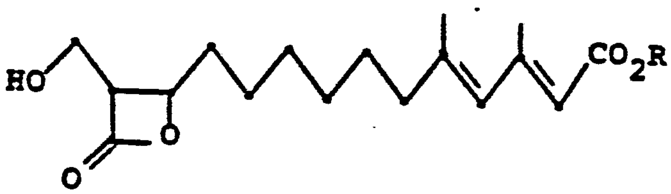

(I)

wherein R is hydrogen or C $_{1\text{-}3}$alkyl; the tetrahydro-analogs thereof; and pharmaceutically acceptable salts thereof. Specifically the compounds of this invention are useful as antifungal agents against pathogenic fungi systemic to and upon the exterior of humans and animals; against agricultural fungi affecting living plants; and against surface fungi on inanimate objects. They may be administered to humans and animals topically, orally or parenterally in the form of a medicated liquid, paste or powder, a capsule, a tablet, an injectable preparation or the like. It is usually desirable to use the oral route. Formulations containing the active ingredient or ingredients and typical inert ingredients are readily prepared. Doses may be varied, depending on the age, severity, body weight and other conditions of human patients but daily dosage for adults is within a range of from about I0 mg/kg to 500 mg/kg (preferably 25 to I00 mg/kg) which may be given in two to four divided doses. Higher doses may be favorably employed as required. Topically, the antifungal agents may be administered at a dosage of from 0.25 to 5 percent of a suitable topical preparation.

The instant compounds are useful against pathogenic mycotic infections upon the surface of or systemic within the body of living animals such as fungi of the Trichophyton spp. species, Cryptococcus spp., Hormodendrum spp., Geotrichum spp., Candida spp., and the like.

The pharmaceutically acceptable salts of the compounds of this invention include those formed from cations such as sodium, potassium, aluminum, calcium, lithium, magnesium, zinc, and from bases such as ammonia, ethylenediamine, N-methyl-glucamine, lysine, arginine, ornithine, choline, N,N′-dibenzylethylenediamine, chloroprocaine, diethanolamine, procaine, N-benzylphenethylamine, diethylamine, piperazine, tris(hydroxymethyl)aminomethane, and tetramethylammonium hydroxide.

As used in the instant invention "fungicide" is intended to include controlling fungi broadly so as to include the killing of fungi as well as inhibiting the growth of fungi.

Fields of technology adversely affected by the lack of effective fungicides are many and include the paint, wood, textile, cosmetic, leather, tobacco, fur, rope, paper, pulp, plastics, fuel, rubber and food industries. Fungicides are also utilized for agricultural application, for instance in preventing or minimizing fungus growth on plants, fruits, seeds or soil.

Although many antifungal agents have been described and used heretofore in an effort to control fungi, none are entirely satisfactory and continued losses resulting from fungal attack make the problem of control a serious and lasting one. The number of fungicides practically useful in combatting fungal growth have been small and only in a few cases have synthetic organic chemicals been found applicable.

The compounds of the invention are effective in controlling the growth of Aspergillus species, for example A. niger , A. flavus, A. fumigatus, A. oryzáe, A. luchensis, A. versicolor, A. sydowi, A. nidulans, A. flaucus and A. terreus, Penicillium species, for example, P. notatum, P. roqueforti, P. chrysogenum , P. oxalicum, P. spinulosum, P. martensii, P. citrinium, P. digitatum, P. expansum, P. italicum, P. cyclopium , and P. funiculosum, Neurospora species such as N. sitophila, Phoma species such as P. terrestrius, Rhizopus species, Alternaria species such as A. solani, Chaetomium species such as C. globosum, Chaetomicum species, for example, C. clivaceum, and Monilia species such as M. sitophila and M. nigra. The above fungi may be found on cosmetics, leather, electrical insulation, textiles, and numerous other materials capable of supporting their growth.

The compounds of this invention may be employed in treatment of plants, soils, fruits, seeds, fur, wood and the like. The fungicidal compounds can be used against soil fungi, such as Rhizoctonia solani, Fusarium solani, and Pythium ultimum , plant fungi, for instance Erysiphe polygoni and Alternaria solani Cochliobolus miyabanus as well as against saprophytes known to attack wood, pulp and lumber such as Lenzites trabea and Ceratocystis pilifera and the fungus Pullularia pullulans which attacks paint.

In particular the compounds of this invention are useful in controlling those agricultural fungus infections that attack growing plants. The compounds are particularly effective against those fungi, which cause rice blast, tomato late blight, tomato early blight, wheat leaf rust, bean powdery mildew and tomato Fusarium wilt.

It should be understood that the compounds may be utilized in diverse formulations, solid, including finely divided powders and granular materials as well as liquid, such as solutions, emulsions, suspensions, concentrates, emulsifiable concentrate, slurries and the like, depending upon the application intended and the formulation media desired.

Thus it will be appreciated that compounds of this invention may be employed to form fungicidally active compositions containing such compounds as essentially active ingredients thereof, which compositions may also include finely divided dry or liquid diluents, extenders, fillers, conditioners and excipients, including various clays, diatomaceous earth, talc, and the like, or water and various organic liquids such as lower alkanols, for example ethanol and isopropanol, or kerosene, benzene, toluene and other petroleum distillate fractions or mixtures thereof.

When the active agents are employed in preventing topical fungal growth, one or more of the compounds may be uniformly distributed in a vehicle that is chemically compatible with the particular compound selected, noninhibiting with respect to the action of the antifungal and essentially non-injurious to body tissue under the conditions of use.

It should be understood that the compounds of the invention may be used in combination, one with the other, as well as with other fungicidally active materials. For instance, a mixture of the active compounds with 2-(4′-thiazolyl)benzimidazole sorbic acid or its salts, propionic acid or its salts, mycostatin, sodium diacetate, trichomycin, amphotericin, griseofulvin, undecylenic acid, chlorquinadol, 5,7-dichloro-8-hydroxyquinoline (Vioform), sodium o-phenylphenate, o-phenylphenol, biphenyl, chlorinated phenols, sodium benzoate, dehydroacetic acid and its salts or esters of parahydroxybenzoic acid, such as the methyl and propyl ester (parabens) can be used to give fungicidal effect when used in appropriate concentrations. It is quite clear, too, that the compounds defined above may be used in conjunction with effective antibacterial materials in appropriate instances so as to combine the action of each in such a situation as to be particularly useful, for instance, in applications where the presence of bacteria creates undesirable results alongside the detrimental action of fungi.

It has been found that growth of various fungi existing in soil is limited or terminated by the addition to the soil of minor quantities of the compounds described. The term soil as used herein is intended to include all media capable of supporting the growth of plants and may include humus, sand, manure, compost, artificially created plant growth solution, and the like.

We have also found that the fungicides of the invention are effective against fungal diseases of plants, and may be effectively used either by direct contact with the foliage or systemically, by introduction through the roots.

The compounds of this invention are conveniently prepared as described in J. Chem. Soc. (C), I971 3888-389I by the fermentation of an identified fungus ACC I233 and the standard chemical transformations disclosed therein. The compound of the formula (I) wherein R is hydrogen may also be prepared by the cultivation of a member of the class of fungi selected from ATCC 20788, ATCC 20789 or ATCC 20790 followed by a standard isolation.

The morphological characteristics of the microorganisms ATCC 20788, ATCC 20789, and ATCC 20790 are described below:

Fusarium sp. MF5045 ATCC 20788

Cultural Characteristics

On Czapek-Dox agar -mycelia is extensive, white and cottony, becoming felted and white with sectors of faint bluish-green tinge or a pale peach tinge as culture ages. Moist areas, faint bluish-green, in color develop where macroconidia are abundant.

On potato-dextrose agar -mycelia is extensive, white and cottony, becoming felted and pinkish-tan in color as culture ages. When vegatative inoculum is used, moist areas, tan in color, develop where macroconidia are abundant.

On Sabouraud-maltose agar -mycelia is extensive, velvety and white with peach to light purple tinge.

Morphological Characteristics

Microconidia are generally unicellular, oval-ellipsoidal, borne singly and held in a gelatinous mass. I.8-2.4$\mu$ $\times$ 3.6 to 4.8$\mu$.

Macroconidia are 3 to 5 celled, thin-walled, ends are tapered and slightly curved. 3.6-4.8$\mu$ $\times$ 24-36$\mu$.

Chlamydospores are abundant, terminal and intercalary, globose, generally smooth-walled, usually formed singly but sometimes formed in pairs.

Fusarium sp. MF5058 ATCC 20789

Cultural Characteristics

On Czapek-Dox agar -mycelia is extensive, white and cottony, becoming felted and white with sectors of a faint bluish-green tinge or a pale peach tinge as culture ages. Moist areas, faint bluish-green in color, develop where macroconidia are abundant.

On potato-dextrose agar -mycelia is extensive, white and cottony, becoming felted and pinkish-tan in color as culture ages. When vegatative inoculum is used, moist areas, tan in color, develop where macroconidia are abundant.

On Sabouraud-maltose agar -mycelia is extensive, velvety and white with peach to light purple tinge.

Morphological Characteristics

Microconidia are generally unicellular, oval-ellipsoidal, borne singly and held in a gelatinous mass. I.8-2.4$\mu$ $\times$ 3.6-4.8$\mu$.

Macroconidia are 3 to 5 celled, thin-walled, ends are tapered and slightly curved 3.6-4.8$\mu$ $\times$ 24-36$\mu$.

Chlamydospores are abundant, terminal and intercalary, globose, generally smooth-walled, usually formed singly but sometimes formed in pairs.

Fusarium sp. MF 5084 ATCC 20790

Cultural Characteristics

On Czapek-Dox agar-mycelia is extensive, white and cottony, becoming felted and white with sectors of a faint bluish-green tinge or a pale peach tinge as culture ages. Moist areas faint bluish-green in color develop where macroconidia are abundant.

On potato-dextrose agar -mycelia is extensive, white and cottony, becoming felted and pinkish-tan in color as culture ages. When vegetative inoculum is used, moist areas, tan in color, develop where macroconidia are abundant.

On Sabourand-maltose agar -mycelia is extensive, velvety and deep-pinkish tan in color. Vegetative growth and medium become purplish-red.

Morphological Characteristics

Microconidia are generally unicellular, oval-elliposoidal, borne singly and held in a gelatinous mass 1.8-2.4$\mu$ $\times$ 3.6-4.8$\mu$.

Macroconidia are 3 to 5 celled, thin-walled, ends are tapered and slightly curved. 3.6-4.8$\mu$ $\times$ 24-36$\mu$.

Chlamydospores are abundant, terminal and intercalary, globose, generally smoothed-walled formed singly but sometime formed in pairs.

The compound of the formula (I) wherein R is hydrogen may be produced by the cultivation of a member of the class of fungi ATCC 20788, ATCC 20789, or ATCC 20790 under the following general conditions.

A preserved source of the culture is used to inoculate an agar slant containing a nutrient medium for growth. After incubation at room temperature for 1 to 5 weeks a portion of this growth is used to inoculate a liquid nutrient medium containing sources of carbon, nitrogen, phosphorus and other elements necessary for life. This medium is incubated at 25 to 30°C, usually 28°. The flask containing the culture and liquid nutrient medium is incubated with or without agitation on a rotary shaker from 0 to 400 RPM, most often at 212 RPM. After 1 to 10 days, when growth is abundant, usually between 2 and 4 days, the culture growth is used to inoculate a flask containing a medium which supports production of the product. Such production media contain carbon sources such as corn, glycerol, corn oil, dextrose, cod oil or peanut meal, nitrogen and sulfur sources such as yeast extract, corn steep liquor, corn, lard water, peanut meal, soy flour, tomato paste and the like as well as organic and inorganic ions such as potassium, phosphorous, calcium, tartrate, iron and magnesium. These production media are inoculated with the culture growth and are incubated at 20 to 30°, most often 25°, for 3 to 30 days, usually 7-14 days with or without agitation.

The compound of the formula (I) wherein R is hydrogen may be recovered from the fermentation medium by extraction with a water miscible solvent, such as $C_{1-3}$ alcohol, especially methanol and water in a ratio from 0.1:1.0 to 1.0 to 0.1, especially 1:1 by volume. The extract is partioned between the aqueous phase and a water miscible organic solvent such as ethyl acetate or methylene chloride. The desired compound is further purified by chromatography on silica gel and then Sephadex LH20 (tradename for dextran derivatives used as gel filtrants in organic solvents, manufactured by Pharmacia Fine Chemicals, Inc.). Finally, the product may be crystallized from aqueous alcohol.

The following examples illustrate the preparation of the compounds and their incorporation into pharmaceutical compositions and as such are not to be construed as limiting the invention set forth in the claims appended hereto.

The composition of media employed in the following examples are listed below. Media are prepared in a 250 ml Erlenmeyer flask. The contents are sterilized with steam at 121°C, 20 pounds pressure for 20 minutes. Media that contain corn are rehydrated and again sterilized with steam at 121°C, 20 pounds pressure for 20 minutes before inoculation.

F867 10 g/flask corn, 0.1 g/flask $MgSO_4.7H_2O$, .01 g/flask $FeSO_4.7H_2O$, 15 ml/flask of (33 g/l yeast extract) after 7 days incubation add 20 ml $H_2O$/flask, incubate without agitation for 14 days

F870 10 g/flask corn, .01 g/flask $FeSO_4.7H_2O$, .01 g/flask $ZnSO_4$. $7H_2O$, 15 ml/flask of (33 g/l yeast extract), incubate without agitation for 14 days

F872 10 g/flask corn, 15 ml/flask of (33 g/l yeast extract) incubate without agitation for 7 days then at 220 rpm for 7 days

F848 10 g/flask corn, 15 ml/flask of ($MgSO_4.7H_2O$ 0.1 g/l, Na tartrate 0.1 g/l, $FeSO_4.7H_2O$ 0.01 g/l, $ZnSO_4.7H_2O$ 0.01 g/l) incubate at 220 rpm for 7 days

KF Corn Steep 5 g
Tomato Paste 40 g
Oat Flour l0 g
Dextrose l0 g
Distilled water l000 ml

Trace Element Mix No. 2-l0ml of ($FeSO_4.7H_2O$ lg/l, $MnSO_4.4H_2O$ lg/l, $CuCl_2.2H_2O$ 25 mg/l, $CaCl_2.2H_2O$ l00 mg/l, $H_3BO_3$ 56mg/l, $(NH_4)_6Mo_7O_{24}.4H_2O$ l9 mg/l, $ZnSO_4.7H_2O$ 200 mg/l Deionized water l000 ml) pH 6.8

YME Yeast extract 4 g
Malt extract l0 g
Dextrose 4 g
Agar 20 g
pH 7
Distilled water l000 ml

EXAMPLE I

Preparation of l2-Hydroxy-l3-hydroxymethyl-3,5,7-trimethyltetradeca-2,4-dienoic acid l2,l4 lactone

(a) Fermentation of ATCC 20790.

A culture of ATCC 20790 was inoculated onto YME slant medium. After growth at 25°C, a portion of this slant was used to inoculate a baffled 250 ml Erlenmeyer flask containing KF growth medium (54 ml). The flask was incubated with agitation at 2l2 rpm on a rotary shaker (2 inch throw) for 3 days at 28°C. Then a portion of the growth (2 ml) was used to inoculate an unbaffled 250 ml Erlenmeyer flask containing Medium F867 (media F870 or F872 may also be employed). After l4 days incubation at 25°C methanol (20 ml) was added and then let stand overnight. Then water (l0 ml) was added to the flask. The desired product was contained in the aqueous methanol extract.

(b) Fermentation of ATCC 20789.

(l) A preserved culture source of ATCC 20789 was used to inoculate a baffled 250 ml Erlenmeyer containing KF growth medium (54 ml). The flask was incubated with agitation at 2l2 rpm on a rotary shaker (2 inch throw) for 3 days at 28°C. Then a portion of the growth (2 ml) was used to inoculate an unbaffled 250 ml Erlenmeyer flask containing medium F870. After l4 days incubation at 25°C, 50 percent aqueous methanol was added. The aqueous methanol extract was employed in the isolation and purification procedure described below.

(c) Fermentation of ATCC 20788.

A culture of ATCC 20788 was used to inoculate a baffled 250 ml Erlenmeyer flask containing KF growth medium (54 ml). The flask was incubated with agitation at 2l2 rpm on a rotary shaker (2 inch throw) for 3 days at 28°C. Then a portion of the growth (2 ml) and remaining medium was used to inoculate an unbaffled 250 ml Erlenmeyer flask containing medium F848. After 7 days incubation at 25°C. the contents of the flask were extracted with methanol (20 ml) in water (l0 ml) to obtain the desired product.

(d) Isolation and Purification

The aqueous methanol extracts (700 ml each) from the fermentation medium from two series of fermentations of the microorganism ATCC 20789 according to the general procedure of Example I(b) were filtered. The first extract was partitioned with methylene chloride (700 ml) and the second extract was partitioned with ethyl acetate (700 ml). In both cases activity was located in the organic phase. The two organic phases were combined and concentrated in vacuo. The residue was dissolved in 4:6 ethyl acetate/hexane (2 ml) and chromatographed on silica gel (200 ml) eluted with a step gradient of ethyl acetate:hexane (4:6, 6:4, 5:5 and 7:3). The desired product was rechromatographed on LH-20 (20 ml) eluted with methylene chloride:hexane: methanol (l0:l0:l) and the desired product eluted from the column with methanol. An analytically pure sample of the title compound was a crystalline compound m.p. 76-77°C.

The $^{13}C$ NMR spectrum was recorded in $CD_3OD$ at ambient room temperature ( 25 mg/0.4 ml) on a Varian XL 400. Chemical shifts are given in ppm downfield of tetramethylsilane relative to the solvent peak at 49.0 ppm as standard. In agreement with the mass spectral data, l8 carbon atoms are observed with the following chemical shifts: l8.5, l9.8 (2X), 26.4, 27.8, 32.0, 35.0, 37.8, 49.9, 58.0, 60.0, 76.3, ll8.7, l30.6, l42.3, l55.7, l70.4, l7l.9 ppm.

Mass Spectrum Calc'd for $C_{18}H_{28}O_5 + H$:
325.2015.
Found 325.2015.

EXAMPLE 2

Preparation of Methyl 12-hydroxy-13-hydroxymethyl-3,5,7-trimethyltetradeca-2,4-dienoic acid 12,14 lactone

To a stirred solution of the compound from Example 1(d) (3.2 mg, 0.01 mmol) in anhydrous diethyl ether (0.5 ml) at ambient temperature, diazomethane dissolved in anhydrous diethyl ether was added dropwise, until the reaction mixture maintained a bright yellow color. The excess diazomethane was removed by bubbling nitrogen through the reaction mixture. The desired product was purified by preparative thin layer chromatography on silica gel eluted with 1 percent methanol in methylene chloride to afford an oil.

IR spectrum max 3520, 1820, 1705 $cm^{-1}$
Mass Spectrum Calc'd for $C_{19}H_{30}O_5$
338.
Found: 338.

EXAMPLE 3

Preparation of 12-Hydroxy-13-hydroxymethyl-3,5,7-trimethyltetradecanoic acid 12,14 lactone

To a solution of the compound from Example 1(d) (32 mg, 0.01 mmol) in glacial acetic acid (0.5 ml) was added platinum oxide (2 mg) and the compound was hydrogenated at ambient temperature under atmospheric pressure. After one hour, the flask was flushed with nitrogen and the reaction mixture filtered. The filtrate was concentrated in vacuo . The residue was purified by preparative thin layer chromatography on silica gel eluted with 3 percent methanol in methylene chloride to afford the above filtered compound as an oil.

IR spectrum max 1820, 1710 $cm^{-1}$
Mass spectrum Calc'd for $C_{18}H_{30}O_4$
(M-$H_2O$):
310.
Found: 310.

EXAMPLE 4

Preparation of Alkali and Alkaline Earth Salts of Compound I wherein R is hydrogen

To a solution of the lactone from Example 1 (42 mg) in ethanol (2 ml) is added aqueous NaOH (1 equivalent). After one hour at room temperature, the mixture is taken to dryness in vacuo to yield the sodium salt of Compound I, wherein R is hydrogen.

In like manner, the potassium salt is prepared using one equivalent of potassium hydroxide, and the calcium salt using one equivalent of CaO.

EXAMPLE 5

As a specific embodiment of an oral composition of a compound of this invention, 20 mg of the lactone from Example 1 is formulated with sufficient finely divided lactose to provide a total amount of 580 to 590 mg to fill a size 0 hard gelatin capsule.

EXAMPLE 6

As a specific embodiment of a parenteral composition of a compound of this invention, 20 mg of the lactone from Example I, as the sodium salt, is dissolved in sterile water, buffered to a pH of 7 with I.0 mM potassium phosphate buffer solution to a concentration of 2.0 percent and is placed in a sterile ampul for parenteral administration.

## Claims

The use of a compound of formula (I).

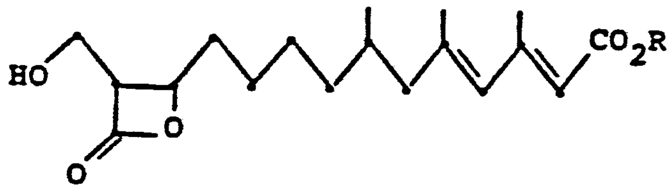

(I)

wherein R is hydrogen of $C_{1-3}$ alkyl;
a tetrahydro analog thereof; or
a pharmaceutically acceptable salt thereof;
for the preparation of a medicament useful for inhibiting fungal growth.

2. The use as claimed in Claim I wherein the fungus to be controlled is within or upon a living animal.

3. The use as claimed in Claim I wherein the fungus to be controlled is upon a plant.

4. The use as claimed in Claim 1 in which the active ingredient is administered with a pharmaceutical carrier.

5. A method of inhibiting fungal growth upon an inanimate object, which comprises the administration upon the object of a compound of formula (I) as defined in Claim I.

6. An antifungal composition comprising an inert carrier and a compound represented by the following general structural formula (I)

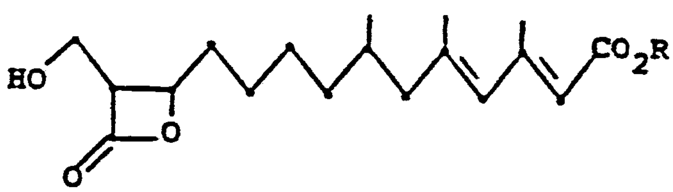

(I)

wherein R is hydrogen or $C_{1-3}$ alkyl;
a tetrahydro analog thereof; or
a pharmaceutically acceptable salt thereof.

7. The composition of Claim 6 which is an oral or parenteral formulation for administration to living animals.

8. The composition of Claim 6 which is a topical formulation for administration to the surfaces of living animals.

9. The composition of Claim 6 which is a solid or liquid formulation for application to agricultural plants or crops.

I0. The composition of Claim 6 which is a solid or liquid formulation for application to inanimate surfaces.

European Patent Office

# EUROPEAN SEARCH REPORT

Application number: EP 87300733.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,X | JOURNAL OF THE CHEMICAL SOCIETY, section C, part III, pages 2739-4002, 1971, London<br>D.C.ALDRIDGE et al. "Antibiotic 1233A: a Fungal β-Lactone" pages 3888-3891<br>* Page 3888, left column; especially formula (1); title; abstract *<br>-- | 1-10 | A 61 K 31/365<br>A 01 N 43/20<br>//C 07 D 305/12 |
| D,X | CHEMICAL COMMUNICATIONS, no. 1, 1970<br>D.C.ALDRIDGE et al. "Anibiotic 1233A: a Fungal β-Lactone"<br>* Page 639, left column; especially formula (1) *<br>---- | 1-10 | |
| | | | **TECHNICAL FIELDS SEARCHED (Int. Cl 4)** |
| | | | A 61 K 31/00<br>A 01 N 43/00<br>C 07 D 305/00<br>C 12 P 17/00 |

The present search report has been drawn up for all claims

| Place of search | Date of completion of the search | Examiner |
|---|---|---|
| VIENNA | 11-05-1987 | MAZZUCCO |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document